(19) Europäisches Patentamt
European Patent Office
Office européen des brevets

(11) Publication number: 0 014 032 B1

(12) # EUROPEAN PATENT SPECIFICATION

(45) Date of publication of patent specification: 23.03.83

(51) Int. Cl.³: C 07 C 125/065

(21) Application number: 80300043.9

(22) Date of filing: 04.01.80

(54) **Process for the preparation of iodoalkynyl carbamates.**

(30) Priority: 22.01.79 GB 7902197
25.05.79 GB 7918319

(43) Date of publication of application:
06.08.80 Bulletin 80/16

(45) Publication of the grant of the patent:
23.03.83 Bulletin 83/12

(84) Designated Contracting States:
BE CH DE FR GB IT NL

(56) References cited:
CHEMICAL ABSTRACTS, vol. 71, no. 1, July 7, 1969, page 286, abstract 3128z. Columbus, Ohio, USA

The file contains technical information submitted after the application was filed and not included in this specification

(73) Proprietor: IMPERIAL CHEMICAL INDUSTRIES PLC
Imperial Chemical House Millbank
London SW1P 3JF (GB)

(72) Inventor: Brand, Boris Peter
12 Elmsway
Bramhall Cheshire, SK7 2AE (GB)
Inventor: Ibbotson, Arthur
29 Simmondley Road
Glossop Derbyshire, SK13 9LP (GB)
Inventor: Gazzard, Edward George
291 Withington Road
Chorlton-cum-Hardy Manchester, M21 1YA (GB)

(74) Representative: Pugsley, Roger Graham et al,
IMPERIAL CHEMICAL INDUSTRIES PLC Legal Department: Patents Thames House North Millbank
London SW1P 4QG (GB)

EP 0 014 032 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Process for the preparation of iodoalkynyl carbamates

This invention relates to a chemical process and more particularly to a process for the manufacture of monoiodoalkynyl carbamates.

US Patent Specification No. 3923870 discloses compounds having the formula:

$$[IC{\equiv}C{-}(CH_2)_n{-}O{-}CONH]_mR$$

Formula I

wherein R is a substituted or unsubstituted alkyl, aryl or alkylaryl group having from 1 to 20 carbon atoms and having from one to three linkages corresponding to *m*, and *m* and *n* are whole number integers between 1 and 3 and may be the same or different. These compounds have fungicidal activity, and are particularly useful as paint film fungicides. The only method disclosed for their preparation is iodination of an alkynol of formula $HC{\equiv}C.(CH_2)_n{-}OH$ to give $IC{\equiv}C.(CH_2)_n{-}OH$, followed by reaction of the latter with an isocyanate of formula $R(NCO)_m$, wherein R, *m* and *n* have the previously defined meanings.

This process has the disadvantages that many of the intermediate iodoalkynols of formula $IC{\equiv}C.(CH_2)_n{-}OH$ are highly vesicant and also tend to be mildly explosive. These properties make manufacture by this route both unpleasant and potentially dangerous.

The formation of the intermediate iodoalkynols can be avoided by reversing the process of USP 3923870, i.e. by reacting the alkynol with an isocyanate to form a carbamate and iodinating the carbamate. It is known from Russian Patent No. 230119 (Chem. Abs. *71*, 1969, 3128z) to iodinate a propargyl arylcarbamate by reaction with $Cu_2Cl_2$ in an ammonia medium and subsequently with iodine in an organic ether solvent. Because of the nature of these reactants and solvents this process is not convenient for commercial operation and it is therefore highly desirable to find an alternative process which can preferably be operated in an aqueous medium. It is has now been found that iodopropargyl carbamates can be iodinated in an aqueous medium with the addition of either an auxiliary solvent or a surfactant to promote contact between the reactants.

According to the present invention there is provided a process for the preparation of a compound of the formula:

$$[IC{\equiv}C{-}(CH_2)_n{-}O.CONH]_mR$$

Formula I

wherein R is a substituted or unsubstituted alkyl, cycloalkyl, aryl, aralkyl or alkylaryl group having from 1 to 20 carbon atoms and having *m* linkages and *m* and *n* are independent integers from 1 to 3, which comprises reacting an alkynol having the formula:

$$HC{\equiv}C.(CH_2)_n{-}OH$$

Formula II

with an isocyanate having the formula $R.(NCO)_m$ to give a carbamate having the formula:

$$[HC{\equiv}C(CH_2)_n{-}O.COnH]_mR$$

Formula III

followed by reaction of the alkynyl carbamate of Formula III with an iodinating agent in an aqueous medium in the presence of an auxiliary water-miscible organic solvent or a surfactant.

The alkynols of Formula II in which *n* is 1, 2 or 3 are known compounds. Prop - 1 - yn - 3 - yl alcohol and but - 1 - yn - 4 - ol are mentioned in Volume 1 of Beilstein's Handbuch der Organischen Chemie at pages 454 and 455 respectively. Pent - 1 - yn - 5 - ol is disclosed in Chemical Abstracts, Vol. 26, page 2166 (1932).

As examples of suitable isocyanates of the formula $R(NCO)_m$ there may be mentioned alkyl isocyanates, such as octadecyl, dodecyl, octyl, hexyl and especially lower alkyl isocyanates such as butyl, propyl, ethyl and methyl isocyanates and the various structural isomers thereof, cycloalkyl isocyanates, such as cyclohexyl isocyanate and monocyclic aryl isocyanates such as phenyl, 4 - chlorophenyl and 3,4 - dichlorophenyl isocyanates in which m is 1 and alkylene diisocyanates such as hexamethylene diisocyanate, arylene diisocyanates such as methylene bisphenyl diisocyanate and the separate or mixed isomers of tolylene diisocyanate and alicyclic diisocyanates such as isophorone diisocyanate in which m is 2.

As examples of the optional substituents that may be present on the above mentioned isocyanates there may be mentioned halogens, such as chloro-, bromo and fluoro and lower, i.e. $C_1$ to $C_4$, alkyl groups.

The first stage of this process, reaction of an alkynol of Formula II with an isocyanate $R.(NCO)_m$, is carried out by methods which are well known in the urethane art. Thus, the isocyanate may be added to the alkynol at a rate such that the reaction temperature is readily controllable, if desired with the aid of external cooling. The reaction may be carried out in the absence of a solvent, or a solvent which is inert to isocyanate groups may be used. Examples of suitable solvents are esters such as ethyl acetate, butyl acetate and 2 - ethoxyethyl acetate, ketones such as methyl ethyl ketone, methyl isobutyl ketone, cyclohexanone and 4 - methoxy - 4 - methylpentan - 2 - one, hydrocarbons such as toluene and xylene, halogenated hydrocarbons such as trichloroethylene, methylene chloride, 1,1,1 - trichloroethane and tetrachloroethane, and polar aprotic

solvents such as dimethylformamide and dimethylacetamide.

There may also be used a catalyst of the kind which accelerates reaction between an isocyanate group and a hydroxyl group. As examples of such catalysts there may be mentioned organic and inorganic basic compounds, and soluble compounds of metals, for example, of transition metals, such as iron and manganese acetylacetonate, and of tin and antimony, for example, dibutyl tin dilaurate and stannous octoate; compounds of lead such as lead acetate, basic lead acetate and lead 2 - ethylhexoate. As basic organic catalysts tertiary amines are suitable, particularly triethylamine, 4 - dimethylaminopyridine, triethylenediamine, dimethylbenzylamine, and dimethylcyclohexylamine. Particularly advantageous are mixtures of metallic and amine catalysts. The amount of catalyst used will depend upon its activity, the temperature used and the rate of reaction desired, but in general an amount from 0.02 to 1.0% by weight, based on the weight of alkynol, will be appropriate. The reaction is conveniently initiated at normal room temperature, e.g. 15 to 25°C, or at a moderately elevated temperature up to 40°C, the temperature preferably being raised to 50—60°C or somewhat higher, e.g. up to 100°C, to promote completion of the reaction. The alkynylcarbamate is isolated from the reaction mixture by conventional means.

The second stage of the process, iodination of the alkynyl carbamate is carried out by reaction of the latter in aqueous medium with an iodinating agent, such as a mixture of sodium hypochlorite and an alkali metal iodide, or a mixture of sodium hypochlorite, an alkali metal hydroxide and iodine, in the presence of an auxiliary water-miscible solvent or a surfactant. A slight molar excess of the iodinating agent is preferably employed to favour complete conversion of the alkynylcarbamate into its iodo derivative.

In order to promote contact between the sparingly soluble alkynyl carbamate and the iodinating agent, the reaction is performed in the presence of an auxiliary solvent, preferably organic, for the alkynyl carbamate which is also miscible with water. As examples of such solvents there may be mentioned alkanols such as methanol and ethanol. The alkynyl carbamate is alternatively dispersed or emulsified in the aqueous medium with the aid of a surfactant so as to maintain it in a finely divided state throughout the reaction period and thereby to promote contact with the iodinating agent.

Classes of substances suitable for use as a surfactant during iodination are protective colloids, such as hydrolysed or partially hydrolysed polyvinyl acetates, phase transfer catalysts such as cetyl trimethyl ammonium bromide, dispersing agents such as the sodium salt of a condensate of formaldehyde and naphthalene sulphonic acid, and polyglycerol ricinoleate, wetting agents such as the sodium salt of di - isopropylnaphthalene sulphonic acid and emulsifying agents such as ethoxylated nonyl phenols.

The effect of the surfactant may be improved by the use of a high speed agitator in the reaction vessel and preferably one which provides some shear action, such as a Silverson or a Turrax mixer. (Silverson and Turrax are trade names).

The temperature during the second stage of the process is preferably maintained at or below 10°C.

The product, i.e. the compound of Formula I, is conveniently isolated by neutralising the reaction mixture with an acid, such as hydrochloric acid, followed by extraction of the iodoalkynyl carbamate from the reaction mixture with a water-immiscible solvent, such as toluene. The product may then be separated from the solvent and used as such or the product may be used as a solution in the solvent as such or after the additon of an emulsifying agent to make the solution self-emulsifiable. Alternatively the product may be dispersed in water or some other medium in which it is substantially insoluble with the aid of appropriate surfactants.

The invention is illustrated by the following Examples in which parts and percentages are by weight.

Example 1

(a) Preparation of prop - 1 - yn - 3 - yl N - n - butylcarbamate

Prop - 1 - yn - 3 - yl alcohol (56 parts), toluene (250 parts), triethylamine (0.257 part) and dibutyl tin dilaurate (0.257 part) are charged to a stirred reaction vessel and *n* - butyl isocyanate (99 parts) is added slowly and continuously during 1 hour. The reaction temperature is controlled between 22° and 37°C by intermittent external cooling. The reaction mixture is then heated to 50°C for six hours, and cooled.

The toluene solution is extracted with 10% sodium carbonate solution (50 parts) and then washed alkali-free with water. The toluene is then evaporated under vacuum at 30°C to leave a quantitative yield of prop - 1 - yn - 3 - yl N - *n* - butylcarbamate as a pale yellow liquid. The infra-red spectrum of this product shows bands at 3320 $cm^{-1}$, 2135 $cm^{-1}$ and 1710 $cm^{-1}$.

(b) Preparation of 1 - iodoprop - 1 - yn - 3 - yl N - n - butylcarbamate

Ethanol (60 parts), water (25 parts) and prop - 1 - yn - 3 - yl N - *n* - butylcarbamate (7.75 parts) are charged to a stirred reaction vessel, followed by sodium hydroxide (3 parts) dissolved in the minimum amount of water The solution is cooled to ≤10°C and iodine (7 parts), followed by sodium hypochlorite (20.5 parts, as a 6% aqueous solution) is

added, maintaining the temperature at ≤10°C. The solution is stirred at this temperature and hydrochloric acid is added to adjust the pH to 7. After allowing the solution to warm to room temperature it is extracted with several portions of toluene. The toluene extracts are combined and dried over anhydrous magnesium sulphate, and the toluene is then removed by evaporation under vacuum to yield 1 - iodoprop - 1 - yn - 3 - yl. N - *n* - butylcarbamate (8.56 parts), m.p. 65—67°C. The N - *n* - infra-red spectrum of this product shows bands at 3340 $cm^{-1}$, 2200 $cm^{-1}$ and 1705 $cm^{-1}$.

Example 2

Ethanol (60 parts), water (25 parts) and prop - 1 - yn - 3 - yl N - *n* - butylcarbamate (7.75 parts) are charged to a stirred reaction vessel followed by sodium iodide (8.25 parts). The resulting solution is cooled to ≤10°C, and sodium hydroxide (2 parts) and sodium hypochlorite (4.7 parts, as a 6% aqueous solution) are added at such a rate as to maintain this temperature. After the reaction mixture has been stirred at ≤10°C for 5 hours sodium bisulphite (5.57 parts as a 40% aqueous solution) is added and the mixture is then neutralised with hydrochloric acid. The solution is extracted with portions of toluene, the toluene extracts are combined and dried over anhydrous magnesium sulphate, and the toluene is removed by evaporation under reduced pressure to yield 1 - iodoprop - 1 - yn - 3 - yl N - *n* - butylcarbamate (8.7 parts).

Example 3

Prop - 1 - yn - 3 - yl N - *n* - butylcarbamate (7.75 parts) and methanol (80 parts) are charged to a stirred reaction vessel and sodium hydroxide (2.89 parts) dissolved in the minimum amount of water is added. The solution is cooled to ≤10°C and iodine (13.33 parts) is added portion-wise during 20 minutes, maintaining the temperature at ≤10°C. The solution is maintained at this temperature for a further 40 minutes before being allowed to rise to room temperature. After 5 hours the solution is extracted with several portions of toluene, the toluene extracts are combined and dried over anhydrous magnesium sulphate, and the toluene is removed by evaporation under reduced pressure to yield 1 - iodoprop - 1 - yn - 3 - yl N - *n* - butylcarbamate (11.2 parts).

Example 4

Prop - 1 - yn - 3 - yl N - *n* - butylcarbamate (15.5 parts) is stirred with sodium hydroxide (0.8 part), potassium iodide (15.5 parts) and a partially hydrolysed polyvinyl acetate sold under the trade name of Gohsenol GL-03 (0.5 part). Sodium hypochloride (8.94 parts) is added as an 11.5% aqueous solution during 75 minutes with control of the temperature to 1—3°C. The white solid dispersion is stirred for a further 30 minutes then a sample examined by the infra-red spectrometer. The absorption at 2110 $cm^{-1}$ characteristic of a HC≡C— residue is absent. That at 2200 $cm^{-1}$ characteristic of an I—C≡C— unit is present.

Example 5

Prop - 1 - yn - 3 - yl N - *n* - butylcarbamate (24.8 parts) is stirred with water (70 parts), polyglycerol ricinoleate (Dispersol OG) (2.6 parts) and iodine (21.4 parts). Sodium hydroxide (25 parts) is added as a 32% aqueous solution during 30 minutes with control of the temperature at ≤10°C followed by sodium hypochlorite (62 parts) as a 12% aqueous solution, again at ≤10°C. The dispersion is stirred for a further 30 minutes then a sample examined by an infra-red spectrometer. The absorption at 2110 $cm^{-1}$ characteristic of a CH≡CH residue is absent. That at 2200 $cm^{-1}$ characteristic of an I—C≡C unit is present.

**Claims**

1. A process for the preparation of a compound of the formula:

$$[IC{\equiv}C{-}(CH_2)_n{-}O.CONH]_mR$$
Formula I

wherein R is a substituted or unsubstituted alkyl, cycloalkyl, aryl, aralkyl or alkylaryl group having from 1 to 20 carbon atoms and having *m* linkages and *m* and *n* are independent integers from 1 to 3, which comprises reacting an alkynol having the formula:

$$HC{\equiv}C.(CH_2)_n{-}OH$$
Formula II

with an isocyanate having the formula $R.(NCO)_m$ to give a carbamate having the formula:

$$[HC{\equiv}C(CH_2)_n{-}O.CONH]_mR$$
Formula III

followed by reaction of the alkynyl carbamate of Formula III with an iodinanting agent in an aqueous medium in the presence of an auxiliary water-miscible organic solvent or a surfactant.

2. A process according to claim 1 wherein the auxiliary solvent is an alkanol.

3. A process according to claim 1 wherein the surfactant is selected from the group comprising a protective colloid, a phase transfer catalyst, a dispersing agent, a wetting agent and an emulsifying agent.

4. A process according to claim 3 wherein the surfactant is selected from the group comprising hydrolysed or partially hydrolysed polyvinyl acetate, cetyl trimethylammonium bromide, the sodium salt of a condensate of formaldehyde and naphthalene sulphonic acid, polyglycerol ricinoleate, the sodium salt of di - isopropylnaphthalene sulphonic acid and ethoxylated nonylphenol.

## Revendications

1. Procédé de préparation d'un composé de formule:

$$[IC\equiv C-(CH_2)_n-O.CONH]_mR$$
formule I

dans laquelle R est un groupe alkyle, cycloalkyle, aryle, aralkyle ou alkylaryle substitué ou non substitué ayant 1 à 20 atomes de carbone et présentant *m* liaisons et *m* et *n* sont des nombres entiers indépendants ayant des valeurs de 1 à 3, qui consiste à faire réagir un alcynol de formule:

$$HC\equiv C.(CH_2)_n-OH$$
formule II

avec un isocyanate de formule $R.(NCO)_m$ pour former un carbamate de formule:

$$[HC\equiv C(CH_2)_n-O.CONH]_mR$$
formule III

puis à faire réagir le carbamate d'alcynyle de formule III avec un agent d'iodation dans un milieu aqueux en présence d'un solvant organique auxiliaire miscible à l'eau ou d'un surfactant.

2. Procédé suivant la revendication 1, dans lequel le solvent auxiliaire est un alcanol.

3. Procédé suivant la revendication 1, dans lequel le surfactant est choisi entre un colloïde protecteur, un catalyseur de transfert de phase, un agent dispersant, un agent mouillant et un agent émulsionnant.

4. Procédé suivant la revendication 3, dans lequel le surfactant est choisi dans le groupe comprenant l'acétate de polyvinyle hydrolysé ou partiellement hydrolysé, le bromure de cétyltriméthylammonium, le sel de sodium d'un produit de condensation de formaldéhyde et d'acide naphtalènesulfonique, le ricinoléate de polyglycérol, le sel de sodium de l'acide diisopropylnaphtalènesulfonique et le nonylphénol éthoxylé.

## Patentansprüche

1. Verfahren zur Herstellung einer Verbindung der Formel

$$[JC\equiv C-(CH_2)_n-O.CONH]_mR$$
Formel I

worin R für eine substituierte oder unsubstituierte Alkyl-, Cycloalkyl-, Aryl-, Aralkyl- oder Alkylarylgruppe mit 1 bis 20 Kohlenstoffatomen steht und m Bindungen aufweist und m und n unabhängig voneinander für Ganzzahlen von 1 bis 3 stehen, bei welchem ein Alkinol der Formel

$$HC\equiv C.(CH_2)_n-OH$$
Formel II

mit einem Isocyanat der Formel $R.(NCO)_m$ umgesetzt wird, wobei ein Carbamat der Formel

$$[HC\equiv C(CH_2)_n-O.CONH]_mR$$
Formel III

entsteht, worauf das Alkinylcarbamat der Formel III mit einem Jodierungsmittel in einem wässrigen Medium in Gegenwart eines mit Wasser mischbaren organischen Hilfslösungsmittels oder eines oberflächenaktiven Mittels umgesetzt wird.

2. Verfahren nach Anspruch 1, bei welchem das Hilfslösungsmittel aus einem Alkanol besteht.

3. Verfahren nach Anspruch 1, bei welchem des oberflächen-aktive Mittel ausgewählt wird aus der Gruppe, die Schutzkolloide, Phasenübergangskatalysatoren, Dispergiermittel, Netzmittel und Emulgiermittel umfaßt.

4. Verfahren nach Anspruch 3, bei welchem das oberflächenaktive Mittel ausgewählt wird aus der Gruppe, die hydrolisiertes oder teilweise hydrolisiertes Polyvinylacetat, Cetyltrimethylammoniumbromid, das Natriumsalz eines Kondensats aus Formaldehyd und Naphthalinsulfonsäure, Polyglycerinricinoleat, das Natriumsalz von Diisopropylnaphthalinsulfonsäure und äthoxyliertes Nonylphenyl umfaßt.